# EUROPÄISCHE PATENTSCHRIFT

(11) **EP 3 310 400 B1**
(45) Veröffentlichungstag und Bekanntmachung des Hinweises auf die Patenterteilung: **20.03.2019**
(21) Anmeldenummer: 16728337.3
(22) Anmeldetag: 13.06.2016
(51) Int. Cl.: A61L 2/26

(54) **VORRICHTUNG ZUR STERILBEHANDLUNG**
DEVICE FOR STERILE TREATMENT
DISPOSITIF DE TRAITEMENT DE STÉRILISATION

(30) Priorität: 19.06.2015 CH 8862015
(43) Veröffentlichungstag der Anmeldung: 25.04.2018
(73) Patentinhaber: Netzhammer, Eric, 4144 Arlesheim (CH)
(72) Erfinder: Netzhammer, Eric, 4144 Arlesheim (CH)
(74) Vertreter: Braun, André jr.
(86) Internationale Anmeldenummer: PCT/EP2016/063436
(87) Internationale Veröffentlichungsnummer: WO 2016/202716

(56) Entgegenhaltungen:
- EP-A1- 1 769 889
- EP-A1- 2 502 636
- EP-A1- 2 823 828
- EP-A2- 2 881 124
- WO-A1-00/61199
- WO-A1-00/74735
- WO-A1-2016/097265
- CN-A- 102 688 718
- DE-U1- 9 421 818

## Beschreibung

Die Erfindung betrifft eine Vorrichtung zur Sterilbehandlung nach dem Oberbegriff des Anspruchs 1.

Für Transfers in der Steriltechnologie werden bekannter Weise Portsysteme, auch bekannt als "Alfa-Beta-Ports" oder "Rapid Transfer Ports (RTP)" eingesetzt. Mit diesen Systemen können sterilisierte Stoffe oder sterilisierte Teile von einem sterilen Behälter zu einem anderen sterilen Behälter transferiert werden. Behandlungsmaschinen für kleine Teile (z.B. Stopfenbehandlungsmaschinen) werden standardmässig mit einem Beta-Port ausgerüstet. Nach der Behandlung wird die Behandlungsmaschine mit den darin befindlichen kleinen Teilen an eine andere Maschine, normalerweise eine Abfüllmaschine, angedockt welche mit einem Alfa-Port ausgerüstet ist. Alfa- und Beta-Port werden miteinander verbunden, und anschliessend wird die Porttür geöffnet damit der Transfer stattfinden kann.

Im Stand der Technik sind verschiedene Behandlungsmaschinen bekannt, welche mit solchen Beta-Ports ausgerüstet sind. Es sind Systeme bekannt welche oben, d.h. auf der Seite des Behälters, die bei der Behandlung oben ist, zwei Anschlüsse aufweisen, einen Port Anschluss für den späteren Transfer und einen weiteren Anschluss für die Ableitung der Prozessmedien(Dampf / Wasser / Luft / etc.), welche für die Behandlung erforderlich sind

Bei einem weiteren bekannten System weist der Behälter oben zwar nur einen Anschluss auf, der aber nachher wiederum in zwei Anschlüsse gegabelt wird, nämlich einen Beta-Port-Anschluss für den Transfer nach der Behandlung und einen weiteren Anschluss für die Ableitung der Prozessmedien. Alle Systeme verfügen unten über einen Anschluss für die Zuführung der erwähnten Prozessmedien.

Einer von mehreren wesentlichen Nachteilen bekannter Systeme besteht darin, dass während der Behandlung der zu reinigenden und zu sterilisierenden Teile der zum Transfer dienende Beta-Port geschlossen sein muss. Die Folge ist, dass die Prozessmedien gewisse Bereiche am Umfang des Portdeckels nicht erreichen können. Diese Bereiche ("Ring of Concern") sind daher am Ende der Behandlung nicht steril und können beim Transfer die behandelten Teile kontaminieren. Es sind auch Vorrichtungen bekannt, beispielsweise aus EP2881124, bei denen der Beta-Port für die Reinigung geöffnet ist und dessen kritische Bereiche mit gereinigt werden. Dafür werden Behälter, die der Behandlung und dem Transport dienen, für die Reinigung an einer ortsfesten, schwenkbaren Halterung befestigt, die gleichzeitig mit den Zufuhr- und Abfuhrleitungen für die Reinigungsmedien versehen ist.

Nachteilig ist bei dieser Lösung, dass das Einhängen des Behälters in die Halterung und dessen Abnehmen einen erheblichen zusätzlichen Aufwand bedeutet.

Der Erfindung liegt die Aufgabe zugrunde, diese Nachteile zu vermeiden.

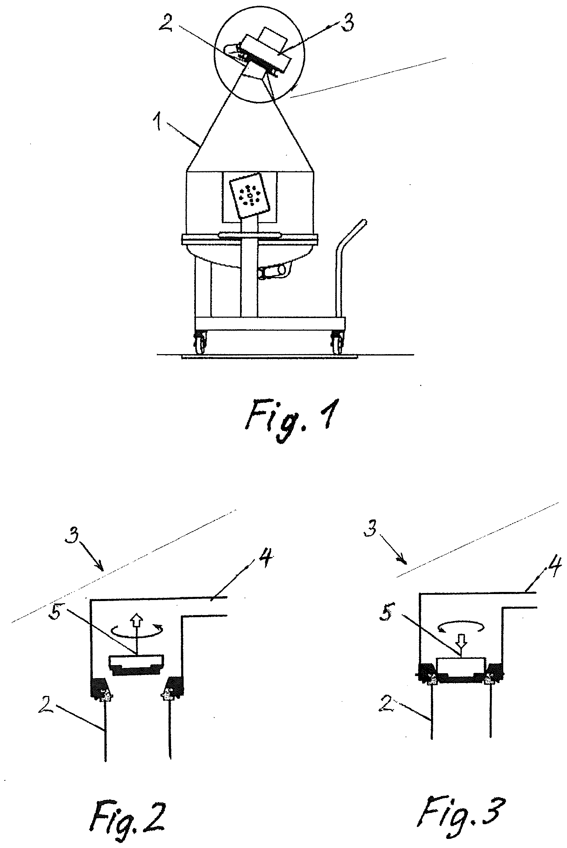

Erfindungsgemäss wird dies erreicht durch die kennzeichnenden Merkmale des Anspruchs 1. Im Folgenden wird anhand der beiliegenden Zeichnungen ein Ausführungsbeispiel der Erfindung beschrieben. Es zeigen
- Fig. 2: eine schematische Schnittdarstellung des Prozessaufsatzes mit geöffnetem Port,
- Fig. 3: eine schematische Schnittdarstellung des Prozessaufsatzes mit geschlossenem Port,
Die vorliegende Beschreibung bezieht sich auf einen Behandlungsbehälter, wie er in der WO 2016/097265 beschrieben ist, d.h. einen Behälter, der fest mit einem Wagen verbunden und auf dem Wagen drehbar angeordnet ist. Wie in Fig. 1 gezeigt, ist der Behandlungsbehälter 1 oben nur noch mit einem Anschluss 2 ohne Verzweigung ausgerüstet, und zwar mit einem Beta-Port, welcher einerseits für den späteren Transfer genutzt wird und andererseits während des Behandlungsprozesses als Durchgang für die Prozessmedien (Dampf / Wasser / Luft / etc.) dient.

Für den Behandlungsprozess wird auf den Port ein Prozessaufsatz 3 aufgesetzt und mittels eines an sich bekannten Klemmsystems, einer Verschraubung oder dergl. dicht mit dem Port verbunden. Der Prozessaufsatz ist seinerseits mit einem Anschluss 4 für die Prozessleitung versehen, durch welche die Prozessmedien abgeleitet werden. Das Klemmsystem kann automatisch oder manuell betätigt sein.

Wie in den Fig. 2 und 3 gezeigt, ist im Inneren des Aufsatzes ein an sich bekanntes Port-Öffnungs-System (POS) 5 angeordnet, mit dem nach dem Aufsetzen des Prozessaufsatzes und dem Anschluss der Prozessleitung der Beta-Port unter Wahrung sämtlicher Sterilanforderungen geöffnet werden kann. Das Portöffnungssystem kann automatisch oder manuell betätigt sein.

Wenn der Portdeckel, wie in Fig. 2 gezeigt, geöffnet ist, ist der Behandlungsbehälter mit der Prozessleitung verbunden und durch die entstehende Öffnung kann der erforderliche Medientransfer stattfinden. Mit dem Durchlauf der Prozessmedien durch den Behälter zur Behandlung der Kleinteile befindet sich auch der Portdeckel und dessen Sitz, also der erwähnte kritische Ring of Concern im Strom der Prozessmedien und wird gereinigt und sterilisiert etc. Nach dem Reinigungs- und Sterilisierungsdurchlauf kann der Portdeckel wieder geschlossen werden, wie in Fig. 3 gezeigt, bis der Behandlungsbehälter an einen anderen Behälter, z.B. den einer Abfüllanlage angedockt wird.

## Patentansprüche

1. Einrichtung zum Reinigen und Sterilisieren in einem Behandlungsbehälter (1), mit einem Produktaustragsanschluss(2) und einem am Austragsanschluss angeordneten Beta-Port, sowie einer Prozessleitung zur Ableitung von Prozessmedien, umfassend einen während der Behandlung mit dem Austragsanschluss und mit der Prozessleitung verbundenen Prozessaufsatz(3) mit einem Portöffnungssystem(5), derart dass die Prozessmedien durch den geöffneten Port strömen und diesen reinigen und sterilisieren,
**dadurch gekennzeichnet, dass**
der Behandlungsbehälter (1) fest mit einem Wagen verbunden ist und auf dem Wagen drehbar angeordnet ist.

2. Reinigungs- und Sterilisierungseinrichtung nach Anspruch 1, **dadurch gekennzeichnet, dass** der Prozessaufsatz(3) einen Innenraum aufweist, durch den die Prozessmedien strömen und in dem das Portöffnungssystem(5) angeordnet ist.

3. Reinigungs- und Sterilisierungseinrichtung nach Anspruch 1, **gekennzeichnet durch** eine Klemmvorrichtung zum dichten Verbinden des Prozessaufsatzes auf dem Austragsanschluss.

4. Reinigungs- und Sterilisierungseinrichtung nach Anspruch 1, **dadurch gekennzeichnet, dass** das Portöffnungssystem(5) automatisch betätigt ist.

5. Reinigungs- und Sterilisierungseinrichtung nach Anspruch 1, **dadurch gekennzeichnet, dass** das Portöffnungssystem(5) manuell betätigbar ist.

6. Reinigungs- und Sterilisierungseinrichtung nach Anspruch 3, **dadurch gekennzeichnet, dass** die Klemmvorrichtung automatisch betätigt ist.

7. Reinigungs- und Sterilisierungseinrichtung nach Anspruch 3, **dadurch gekennzeichnet, dass** die Klemmvorrichtung manuell betätigbar ist.

## Claims

1. Device for cleaning and sterilizing in a treatment container (1), comprising a product discharge connection (2) and a beta port arranged on the discharge connection, and a process line for discharging process media, comprising a process attachment (3) which has a port opening system (5) and is connected to the discharge connection and to the process line during treatment, such that the process media flow through the open port and clean and sterilize said port,
**characterized in that** the treatment container (1) is rigidly connected to a carriage and is rotatably arranged on the carriage.

2. Cleaning and sterilizing device according to claim 1, **characterized in that** the process attachment (3) comprises an interior through which the process media flow and in which the port opening system (5) is arranged.

3. Cleaning and sterilizing device according to claim 1, **characterized by** a clamping means for sealingly connecting the process attachment to the discharge connection.

4. Cleaning and sterilizing device according to claim 1, **characterized in that** the port opening system (5) is operated automatically.

5. Cleaning and sterilizing device according to claim 1, **characterized in that** the port opening system (5) can be operated manually.

6. Cleaning and sterilizing device according to claim 3, **characterized in that** the clamping means is operated automatically.

7. Cleaning and sterilizing device according to claim 3, **characterized in that** the clamping means can be operated manually.

## Revendications

1. Dispositif de nettoyage et de stérilisation placé dans un récipient de traitement (1) muni d'un raccord de sortie de produit (2) et d'un port bêta disposé au niveau du raccord de sortie, ainsi que d'un conduit de processus destiné à évacuer des milieux de processus, le dispositif comprenant un embout de processus (3) qui est relié, pendant le traitement, au raccord de sortie et au conduit de processus et qui comporte un système d'ouverture de port (5) de telle sorte que les milieux de processus s'écoulent à travers le port ouvert et le nettoient et le stérilisent,
**caractérisé en ce que**
le récipient de traitement (1) est relié de manière fixe à un chariot et est monté à rotation sur le chariot.

2. Dispositif de nettoyage et de stérilisation selon la revendication 1, **caractérisé en ce que** l'embout de traitement processus (3) comporte un espace intérieur à travers lequel les milieux de processus s'écoulent et dans lequel le système d'ouverture de port (5) est disposé.

3. Dispositif de nettoyage et de stérilisation selon la revendication 1, **caractérisé par** un dispositif de serrage destiné à relier de manière étanche l'embout de processus au raccord de sortie.

4. Dispositif de nettoyage et de stérilisation selon la revendication 1, **caractérisé en ce que** le système d'ouverture de port (5) est actionné automatiquement.

5. Dispositif de nettoyage et de stérilisation selon la revendication 1, **caractérisé en ce que** le système d'ouverture de port (5) peut être actionné manuellement.

6. Dispositif de nettoyage et de stérilisation selon la revendication 3, **caractérisé en ce que** le dispositif de serrage est actionné automatiquement.

7. Dispositif de nettoyage et de stérilisation selon la revendication 3, **caractérisé en ce que** le dispositif de serrage peut être actionné manuellement.
